# EUROPEAN PATENT APPLICATION

(11) **EP 2 733 486 A1**
(43) Date of publication of application: **21.05.2014**
(21) Application number: 12810738.0
(22) Date of filing: 26.04.2012
(51) Int. Cl.: G01N 33/08

(54) **PORTABLE EGG CANDLER FOR MEASUREMENT**

(30) Priority: 11.07.2011 ES 201131169
(71) Applicant: Ismael, Royo Bieto, 43393 Almoster (Tarragona) (ES)
(72) Inventor: Ismael, Royo Bieto, 43393 Almoster (Tarragona) (ES)
(74) Representative: Carpintero Lopez, Francisco
(86) International application number: PCT/ES2012/070284
(87) International publication number: WO 2013/007843

(57) **Abstract**

An egg candler particularly designed to measure the height of the air chamber inside eggs from chickens or other animal species, in order to determine the degree of freshness and the quality of the egg, is described. The egg candler comprises a structure that provides an inner cavity that encloses a rotatable supporting container with a housing for insertion of the egg, which is operable by means of a manual or motorized transmission mechanism, a laser illumination source that can be positioned manually or by means of a motor in height and closeness to the surface of the egg, even to the point of touching said surface, and a webcam for capturing images and sending said images to a computer linked to said webcam for viewing and accurate evaluation of the magnitude representing the height of the inner air chamber.

## Description

### Object of the Invention

The present invention relates to a portable egg candler for measurement, which provides essential novelty features and considerable advantages with respect to conventional egg candlers in the current state of the art, which only allow observing, with very little resolution, the embryonic development or some characteristics of the inside of the eggs.

More particularly, the invention provides an egg candler consisting of a portable electronic instrument based on a low-cost technique, designed specifically for observing the inside of the eggs sold with a shell, intended for assessing the qualitative characteristics of said eggs and measuring, directly in millimeters and tenths of a millimeter and with a high degree of accuracy, the height of the air chamber which all eggs have inside their most spherical end, for the purpose of determining their degree of freshness and quality from the measurements taken and observations made. The egg candler incorporates a supporting container device with variable positioning, in which the egg is placed with the pointiest end facing downwards, the most spherical portion of the egg being exposed to the action of laser illumination, specifically and as a result of research conducted, a laser with a power of 10 mW with a wavelength comprised between 520 and 560 nanometers, which allows viewing the inner air chamber regardless of the color of the shell and of the thickness thereof, must be used, said supporting container being moved rotationally with the aid of a transmission mechanism. A webcam or other viewing means allows viewing directly, on a computer screen, the area of the egg where the air chamber is located, for the purpose of the evaluation of the height of said air chamber.

The field of application of the invention is comprised in the industrial sector dedicated to manufacturing multipurpose measuring devices and instruments.

### Background and Summary of the Invention

Some devices referred to as "egg candlers" intended for providing information about certain internal characteristics of a chicken egg or of another type of egg are known in the current state of the art. A conventional egg candler consists of a manual instrument made up of a powerful light beam which, upon being projected on an egg, by means of a technique referred to as back light allows certain translucent vision of certain opaque objects (for example, eggs), for observing images of their inside.

These known manual instruments have conventionally been used to identify if there is a live embryo inside a fertile egg and during its incubation process, and to thus enable observing, albeit with very low resolution, its different phases of development and also the possible micro-fissures that the shell of some of them may have.

In contrast, there has been no knowledge to date of being able to use a conventional egg candler as an instrument truly useful for measuring, with the necessary accuracy, the height of the air chamber that all eggs have inside their most spherical end, as well as other qualitative characteristics of said eggs that allow determining the degree of freshness and the quality of the eggs sold with a shell for direct consumption. This is because the aforementioned conventional back light technique has the drawback of deficient resolution of the images that can be observed using said technique, as well as the impossibility of taking any measurement with the necessary accuracy.

Another significant drawback observed in egg candlers based on the back light technique consists of the high generation of heat irradiated by the electric lamps they use. This particularity means that egg candlers must be complemented with temperature diffusing systems and devices, and the alternation of operating periods with disconnect periods for the purpose of being able to maintain a suitable thermal level.

Given that increasingly greater control is required in all manufacturing processes, and quite particularly in relation to food production, the conventional egg candler is insufficient and inadequate for being able to perform, with the necessary accuracy, the mandatory controls required under EC Regulations in relation to the Standards for Food Safety, for all entities that produce and package eggs intended for direct consumption.

Thus, according to the conducted studies, it could be established that in the egg there is a direct, accurate and reliable relationship, which is inversely proportional, between the height of the air chamber, which is located inside its most spherical pole, and the degree of freshness of the egg. In other words, the lower the height of an egg's inner air chamber the fresher it is.

Both this measurement and the observation of the qualitative characteristics of the shell and the cuticle (inner membrane adhered to the shell), the yolk, the egg white and the germ, as well as the possibility of seeing if there is any foreign matter inside the egg, determining if the egg meets the indicated characteristics so that it can be legally sold as Grade A (i.e., for sale with a shell directly to the consumer), or if it must be sold as Grade B (i.e., it must be sold only to industry to be used, after being exposed to a pasteurization treatment or other treatments, in different processes for elaborating various products).

Furthermore, it is advisable and necessary to be able to have an instrument with modern technology that can be connected to a computer and process all the measurements and controls that are performed with it by computing, avoiding the need for manual data recordings that can be inaccurate, erroneous, or be unintentionally modified, all of which will allow being able to establish a suitable and reliable system of complete control and traceability.

Taking the foregoing into account, the proposed main objective of the present invention is the design and construction of an egg candler that overcomes the drawbacks and deficiencies of currently existing egg candlers. This objective has been fully met by means of the egg candler that will be described below, the essential features of which are included in the characterizing part of the attached Claim 1.

Essentially, the portable egg candler for measurement proposed by the present invention consists of a tabletop structure designed with a format similar to that of a small electrical appliance, based on a structure or container with a cover or another hermetic sealing system, so that once the egg is inserted inside it for measurement and assessment of the characteristics sought, said egg is deposited in a completely dark environment.

It is envisaged that the structure can be made with various materials, preferably lightweight and opaque materials, which have a surface that can be washed and disinfected with suitable products without being damaged by said products.

It is envisaged that the structure is portable, for which purpose it can be equipped with a handle with clasps or other similar means located at predetermined points of said structure, for installing a belt that allows carrying said structure hanging from the waist, shoulder or neck of the person responsible for moving it around. However, it must be understood that the portability feature is only one desired particularity, the structure being able to be designed to be fixed to any supporting surface without this involving any alteration of the principles of the present invention.

As operating members, the structure internally includes a supporting container intended for receiving the egg with the pointiest end facing downwards, the egg being fixed and unable to slide; a means constituting a laser illumination source defined at the beginning that can be positioned very closely to the surface of the egg, even to the point of touching the shell, and capacitated for relative displacement to the left-right or variable positioning with respect to the vertical, such that it describes a closed curve according to the meridian of the upper third of the corresponding hemisphere of the egg; a transmission mechanism that acts on the supporting container in which the egg is deposited to make it rotate to the left or to the right, and image capture and evaluation means, such as a webcam operatively connected to a PC-type computer responsible for analyzing the measurements and providing a numerical result, expressed in millimeters and tenths of a millimeter, with respect to the magnitude of the height of the inner chamber.

Both the displacement of the device containing the source for emitting the laser beam and the transmission mechanism of the supporting container in which the egg is deposited can be operated manually or by means of a motor, whichever is appropriate.

Additionally, the egg candler as a whole includes other means necessary for its correct operation, such as electric and/or electronic means, USB connections, power supply, etc.

### Brief Description of the Drawings

The foregoing and other features and advantages of the invention will be understood more clearly from the following detailed description of a preferred embodiment thereof, given only by way of non-limiting illustrative example in reference to the attached drawings, in which:
Figure 1 is a schematic side elevational view of a longitudinal section made in a portable egg candler for measurement built according to the teachings of the present invention;
Figure 2 is a schematic front elevational view of the same egg candler shown in Figure 1 discussed above;
Figure 3 illustrates a sequence of images (a) - (d) that can be seen in the PC-type computer monitor prior to the measurement of the height of the air chamber of an egg positioned inside the supporting container of the egg candler of Figures 1 and 2; and
Figure 4 shows a schematic depiction of an enlarged detail illustrating the final determining image of the height of the air chamber obtained as a final result of the measurement operation performed on an egg positioned inside the egg candler of said Figures 1 and 2 above.

### Description of a Preferred Embodiment

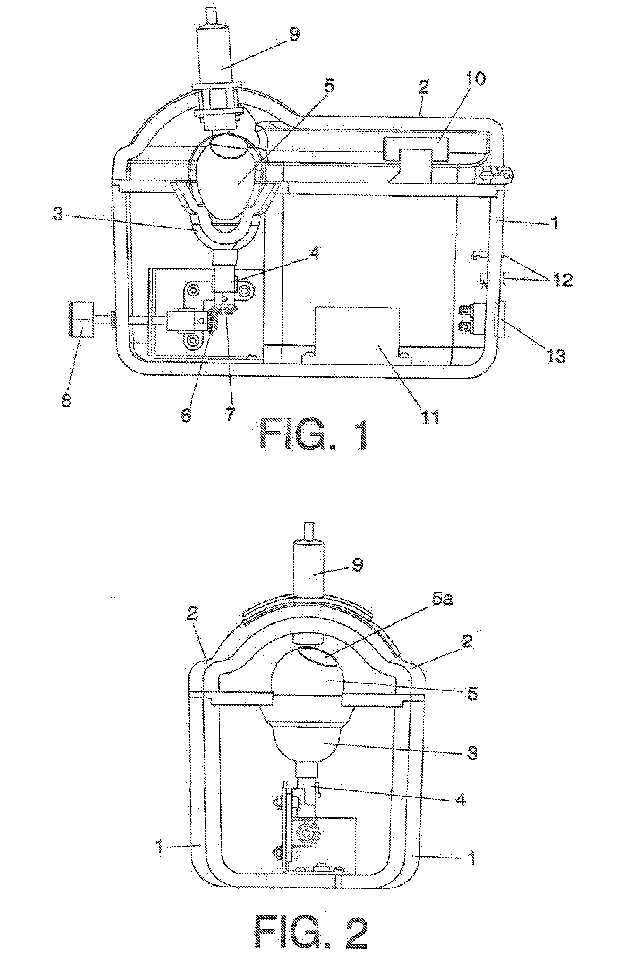

As mentioned above, the detailed description of the preferred embodiment of the object of the invention will be provided below with the aid of the attached drawings in which the same reference numbers are used to designate the same or equivalent parts. Therefore, first considering Figure 1 of the drawings, said figure shows a schematic side elevational depiction of a longitudinal section made in an egg candler of the present invention, obtained from a structure that can be positioned on the surface of a table or the like, made up of a lower base part 1 that provides a cavity for the various operating members integrated in the egg candler, and a closure system for closing said cavity, such as a cover 2 that can be removed to access the inner space of the cavity provided by said lower part 1 of the structure. A supporting container 3, designed such that it allows positioning an egg 5 in a housing of the upper portion thereof with the pointiest end of the egg occupying the lower position, is supported by a rotating shaft 4 capable of rotational operation to the left and to the right by means of a mechanism which, in the depicted case, is operated manually and incorporates two bevel gears 6, 7 mutually coupled to one another, one bevel gear 6 of which can be moved manually from the outside by means of a button 8, but which in other embodiments can be operated by means of a motor.

In the preferred embodiment, the supporting container 3 is detachably coupled to the shaft 4, which provides the added advantage that it can be removed, when appropriate, to be washed and sanitized, or to be replaced if needed. Its upper housing for receiving the egg is capacitated for accommodating eggs of different sizes, both chicken eggs and eggs of other animal species.

The egg 5 deposited in the housing of the supporting container 3 is kept fixed, without the possibility of sliding or of otherwise moving during the measurement-assessment process, such that the measurement of the height of its air chamber can be taken with an accuracy of tenths of a millimeter.

Given the position in which the egg 5 is located, its air chamber is occupying the upper position, i.e., it corresponds with the most spherical end of the egg occupying the highest position. There has been envisaged on this highest portion of the egg the arrangement of a powerful light source, particularly a laser source 9, with a power of 10 mW and a wavelength comprised between 520 and 560 nanometers, which can positionally vary both in relation to its closeness to the surface of the shell of the egg 5 and in relation to its positioning with respect to the vertical. It can therefore be placed very close to the shell of the egg, even to the point of touching said shell, and on the other hand, a position such that by rotating the egg, the light projected on the shell can follow a path corresponding to the meridian that demarcates the upper third of the corresponding hemisphere of the egg, as that is the position occupied by the air chamber the height of which is to be measured. Maximum viewing contrast of the air chamber is achieved when the laser beam strikes the interface between the air chamber and the egg white, a point, as previously stated, which can be reached with the lateral displacement and vertical regulation of the laser source 9.

According to the invention, to obtain maximum efficiency in viewing said air chamber of the eggs 5, it is necessary for the laser source to be located as close as possible, even to the point of touching the shell of the egg and perpendicularly striking said shell. Therefore, viewing the air chamber in any type of egg, regardless of shell color and the thickness of said shell will be the most suitable for taking the desired measurement. According to the invention, a laser source which has been shown to be particularly suitable for use in the egg candler of the present invention is a laser source with a power of 10 mW and a wavelength comprised between 520 and 560 nanometers, providing illumination of the air chamber of the egg that is sufficient for being able to take an accurate measurement of the height of the air chamber and thereby determine the degree of freshness of the egg.

The use of a webcam shown in Figure 1 of the drawings and indicated with reference number 10, which can be positioned opposite said air chamber of the egg 5 in order to capture the corresponding images and sending said images to a computer to be viewed on the computer screen, has been envisaged as the image capture means for capturing images during the aforementioned measurement process. The figure depicts other electrical and/or electronic components such as, for example, a power supply 11, USB 12 connectors or connectors 13 of another type, necessary for normal operation of the egg candler.

Now in reference to Figure 2 of the drawings, a sectioned front elevational view of the egg candler of Figure 1 can be seen. This depiction shows the egg candler with a transverse dimension that is noticeably smaller than the longitudinal dimension, and it allows seeing the area 5a of the most spherical end of the egg 5 inside which the air chamber the height of which is to be measured is located.

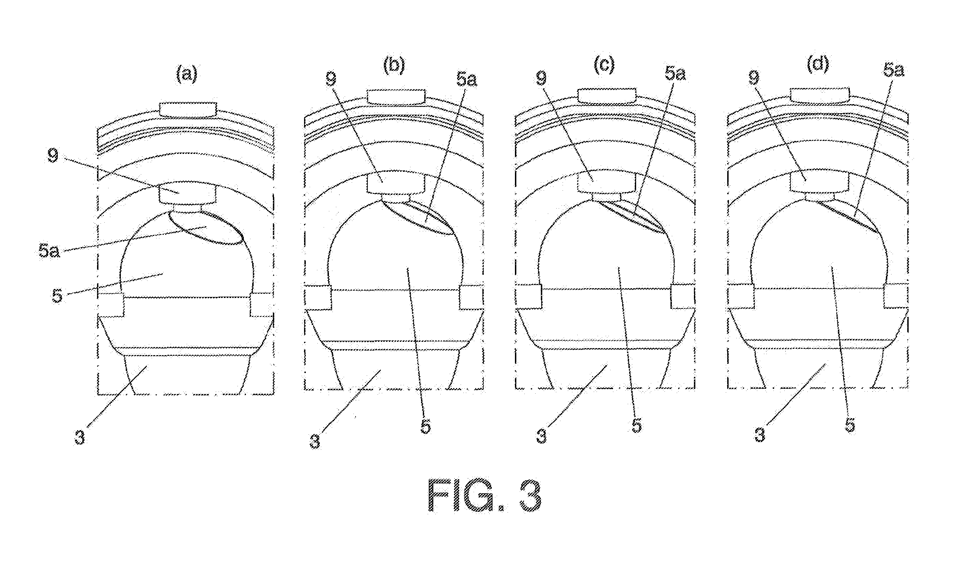

With the constructive features of the egg candler such as those described above, the measurement process for measuring the height of the air chamber located in the upper portion of the egg, behind the surface area indicated as 5a, by measuring the height of the spherical cap defining it, can be carried out simply, quickly and accurately. Figure 3 of the drawings shows a sequence of images referenced as (a) to (d), corresponding to the necessary process of positioning the egg prior to measurement. Therefore, once the egg 5 has been arranged in the upper housing of the supporting container 3 with the most spherical portion located in the upper position, and after the cover 2 is closed on the lower part 1, the webcam 10 is connected and the laser 9 is moved over the most spherical portion of the egg opposite to it, such that when the light penetrates the egg, it illuminates said chamber showing a clear contrast between the area where there is air and the area where the egg white is located, thus determining the exact interface point between them.

Once the position of the illuminated portion of the egg is controlled in a monitor of a PC-type computer (not depicted) receiving data from the webcam 10, it is rotated by means of the motorized or manual transmission system in which said bevel gears 6, 7 are integrated through the button 8, transmitting said rotational movement to the shaft 4, to the left and to the right, and by means of the latter, to the supporting container 3 with the egg 5 therein, successively passing though positions such as those indicated as (a), (b), (c) and (d), such that the surface portion 5a gradually advances at an angle until the plane formed by the base of the illuminated chamber lines up with the webcam and a straight line resulting from the superposition of the projection of the line of sight of its two base lines, as indicated in the last position of the sequence referenced as (d), is seen on the computer screen.

The position of the laser gun 9 may have to be manually or automatically corrected during the rotational movement described for the egg 5, such that said laser continues to correctly illuminate the inside of the air chamber, because since it is normally located with somewhat of an inclination with respect to a virtual vertical axis of the egg, it could vary the position of its interface with rotation.

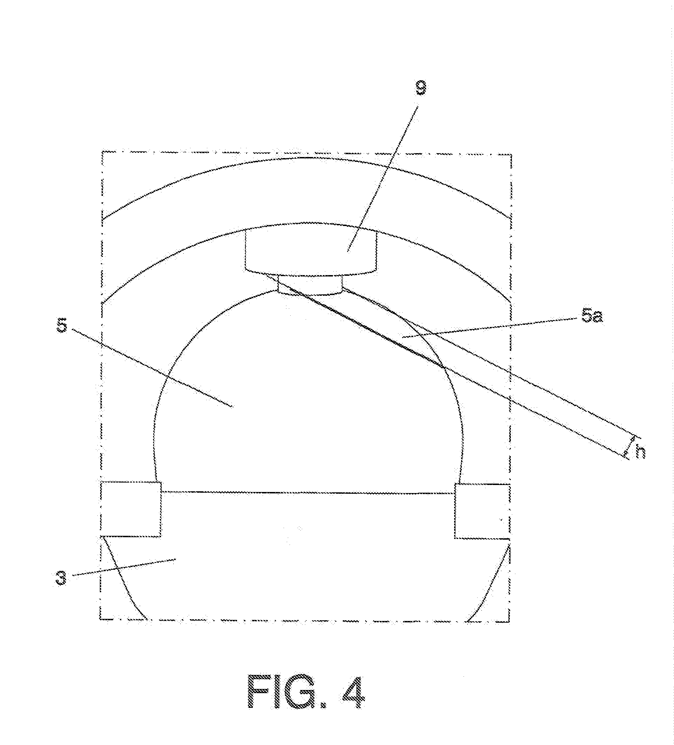

When the base of the air chamber is seen to be straight (position (d)), as shown in the depiction of the enlarged detail in Figure 4 of the drawings, it is finally possible to measure the distance "h" separating the straight line of the area 5a with respect to the shell of the egg as a true magnitude. This measurement of "h" corresponds with the magnitude that is sought.

The value of "h" may be determined with the aid of software designed specifically to determine the distance between two previously indicated points using the computer mouse or by reading it directly on the monitor by means of a graduated rule in said monitor which could be suitably oriented according to the straight line formed by the base of the air chamber being viewed.

Once the result of the measurement is obtained, this value may be stored in a database by means of program or software that is also designed specifically, which allows obtaining a record of all the measurements taken, controlling the day, time, the lot of chickens or other animal species to which each measured egg corresponds, as well as any other information that can be considered relevant and that can allow, in addition to being able to store all those controls indefinitely, obtaining complete traceability throughout the packaging and marketing performed on a food that is as important as eggs between production and consumption.

Finally, as described in another part of the present description, an additional feature is that the portable version of the structure made up of the lower part 1 and the cover 2 can include fixing means (not depicted) for fixing in predetermined positions to allow its engagement by means of belts or the like which facilitate transport by users.

It is not considered necessary to expand the content of the present description so that a person skilled in the art can understand its scope and the advantages derived from the object thereof.

Nevertheless, and since the description given relates only to an embodiment of the object of the invention, it will be understood that, within its essential features, it is possible to introduce many variations in detail, which variations are equally protected and could affect features such as the shape, size or the materials for manufacturing the assembly or its parts, or any other variations that do not alter the essential features of the invention demarcated only by the scope of the claims below.

## Claims

1. Portable egg candler for measurement, particularly an egg candler particularly designed like a small tabletop electrical appliance for measuring the air chamber included in an egg (5) in relation to the shell portion associated with the most spherical end of the egg, to determine the state of freshness and the quality of the egg being examined, **characterized in that** it consists of a structure obtained from a lower part (1) or base that provides a cavity for housing the various operating members of the egg candler, and a cover (2) hermetically sealing said base part (1), inside which there is housed a supporting container device (3) which at the upper end has the formation of a housing intended for accommodating an egg (5) the height of the inner air chamber of which egg is to be measured and other qualitative characteristics of which are to be assessed, positioned with its pointiest end facing downwards, this supporting container (3) being held by a rotating shaft (4) rotating to the left and to the right receiving movement through a transmission mechanism, whereas in the upper closure cover (2), fixed in a position facing the position occupied by said egg (5), the egg candler includes a laser source (9) capable of maximum closeness, even to the point of touching the surface of the portion (5a) of the shell of the egg protecting the inner air chamber, this laser source (9) being able to be positioned with respect to the vertical.

2. Egg candler according to claim 1, **characterized in that** it includes a webcam (10) for taking images of the air chamber and other characteristics of the inside of the egg (5) once illuminated by the laser source (9), and sending such images to a computer linked to said webcam for assessment of the information received, for the display of the images on the corresponding monitor, and the accurate determination of the magnitude ("h") of the height of the inner air chamber with the aid of specific software.

3. Egg candler according to claim 1, **characterized in that** the coupling between the supporting container (3) and the shaft (4) that holds it is separable for the purpose of maintenance and/or replacement of the first mentioned element.

4. Egg candler according to claim 1, **characterized in that** the operation of the transmission mechanism for the rotation of the supporting container (3) can be motorized or manual by means of an outside button (8).

5. Egg candler according to claim 2, **characterized in that** the positioning of the laser source (9) can be performed manually or by means of a motor from the outside.

6. Egg candler according to the preceding claims, **characterized in that** the laser source (9) is a source with a power of 10 mW, and it emits at a wavelength comprised between 520 and 560 nm.
